# EUROPEAN PATENT APPLICATION

(11) **EP 0 953 841 A2**
(43) Date of publication of application: **03.11.1999**
(21) Application number: 99108447.6
(22) Date of filing: 30.04.1999
(51) Int. Cl.: G01N 33/497

(54) **Breath monitoring apparatus**

(30) Priority: 30.04.1998 US 170187
(71) Applicant: Alcohol Sensors International, Inc., Islandia, New York 11722 (US)
(72) Inventor: Koppel, Ronald, Huntington, New York (US); Kirchmeier, Edwin, Hauppauge, New York (US)
(74) Representative: Türk, Gille, Hrabal

(57) **Abstract**

An apparatus for measuring the concentration of a human breath constituent, such as alcohol, comprises a hand-held housing having an internal breath passageway with a first end through a nozzle mounted to the housing. A sensor module for detecting the constituent is mounted in the housing and provides an output signal corresponding to the presence of the constituent to be sensed. A pressure transducer is coupled to the passageway for measuring the pressure therein to allow the validity of the breath sample to be determined. A sonic signalling element is also mounted in the housing for generation of indicator sounds perceptible outside the housing to provide the user with feedback regarding the breath sample applied. Electrical coupling means in said housing couple the sensor module, pressure transducer and sonic signalling element to a remote processor.

## Description

### Background of the Invention

There have been numerous attempts to provide devices to determine the alcohol level of blood, which provides a legal definition for the offenses of driving while intoxicated or driving in an alcohol-impaired state. A common approach employed in such devices is the use of an alcohol-responsive sensor adapted to measure the amount of alcohol in a breath sample to which the sensor is exposed. A sensor output reading corresponding to a given breath alcohol concentration level can be correlated to an associated blood alcohol level, which information can be utilized to lock out an ignition circuit, issue an alarm, or provide other appropriate signal outputs when the sensed alcohol level exceeds a chosen reference value.

The criteria of primary importance for an acceptable sensor system is that the sensor provide an accurate indication of the alcohol level of the breath and that it exhibits a high immunity to false or spurious breaths. The National Highway Traffic Safety Administration has published a Model Specification for breath alcohol ignition interlock devices which attempt to quantify some of the necessary operating parameters for such system. See 57 Fed. Reg. 11772 (#67), April 7, 1992.

Obtaining the accuracy criteria has been illusive. Vapor sensors of the type typically used in alcohol sensors, which vary their resistance in response to the presence of organic vapors, including alcohol vapors, have been found to be subject to random resistance variations due to temperature, humidity, unit-to-unit manufacturing tolerances, and variations in the condition and responsiveness of the reactive surface area resulting from the required repeated cleaning cycles utilized to maintain sensor sensitivity and operability. Yet they remain a sensor of choice because of their availability, low cost, and general ruggedness and ease of use.

The ability to differentiate between a valid human breath and artificial inputs, as well as to identify human breaths which, due to their characteristics, may not provide a proper sample has also been difficult to meet. For example, it is important that the breath sample comprise alveolar air, which most closely tracks blood alcohol level. The breath must be of sufficient duration and depth to offer such air to a sensor system. Complex systems, relying on combinations of temperature, humidity and the like to validate a breath, have often failed to provide sufficient accuracy of identification to insure meaningful results.

### Brief Description of the Invention

It is accordingly a purpose of the present invention to provide a breath sensor apparatus, and particularly a breath alcohol sensor apparatus, which exhibits high reliability in identifying an input as a true human breath, and quantifying the level of a sensed breath component.

It is a further purpose of the present invention to provide a breath sensor apparatus which may be presented in a small and compact housing which may, for example, be easily accommodated in the hand of a user.

Still another purpose of the present invention is to provide a breath sensor apparatus of improved accuracy and of compact and rugged construction which, in one preferred embodiment, avoids the necessity for re-calibration or periodic cleansing cycles.

Yet another purpose of the present invention is to provide a breath sensor apparatus which provides a feedback signal to the user to assist the user in providing a usable breath sample.

In accordance with the foregoing and other purposes, a breath sensor unit of the present invention incorporates a sensor module which may be of various constructions, including those which utilize optical characteristics sensitive to the presence of a particular substance, such as ethanol, in a sample to generate an electrical output signal. Semiconductor-type gas sensors may also be used. In general, a characteristic of the sensor module's output is proportional to the concentration of the sensed substance. The sensing module is located in a small, lightweight housing having an input air passageway. A first branch of the passageway leads to the sensor module. A second branch of the passageway within the housing leads to a pressure transducer. The outputs of the sensor module and pressure transducer are coupled to appropriate analysis and processing circuitry located remote from the housing. The housing further includes a sonic signalling device coupled to the processing circuitry to provide instructional cues to the user for operation of the breath sensor unit.

### Brief Description of the Drawings

A fuller understanding of the present invention will be obtained upon consideration of the following detailed description of a preferred, but nonetheless illustrative, embodiment of the invention when reviewed in association with the annexed drawings, wherein:
FIG. 1 is a plan view of a hand-held breath sensor unit of the present invention;
FIG. 2 is a pictorial representation of the orientation of the functional elements of a first embodiment of the invention within the housing;
FIG. 3 is a schematic representation of the functional elements of the first embodiment of the invention;
FIG. 4 is a schematic representation of a second embodiment of the invention;
FIG. 5 is a pictorial representation of the second embodiment;
FIG. 6 is a side elevation view of the sensor module construction of the second embodiment;
FIG. 7 is a plan view thereof; and
FIG. 8 is a block diagram of the invention and associated system elements.

### Detailed Description of the Invention

The present invention incorporates the use of a sensor responsive to the presence of a particular sample constituent, and in particular ethyl alcohol, in the breath of a user. A first preferred sensor is a fiber optic element in the form of a waveguide which directs and divides a beam of light between and along sampling and reference paths within the waveguide. The sampling path of the waveguide is provided with an optical coating, the characteristics of which vary as it interacts with the constituent of interest in the sample, thus affecting the light transmission properties of the waveguide. By comparison of the sampling and reference light beams a determination can be made as to the concentration of the constituent to which the sampling waveguide has been exposed. Such sensors are preferably constructed in accordance with the teachings of U.S. Patent Nos. 5,439,947 for a Chip Level Waveguide Sensor and U.S. Patent No. 4,846,548 for Fiber Optic Which Is An Inherent Chemical Sensor.

A waveguide sensor of the aforementioned type may be further fabricated as known in the art into a module with appropriate semiconductor circuitry to both drive the sensor and generate and condition appropriate output signals. Such circuitry, as known in the art, and as may be provided by Texas Instruments of Dallas, Texas may incorporate voltage-to-frequency convertors in which sampled and reference output signals generated by optoelectrical elements take the form of varying frequency wavetrains, the frequencies of which may be compared to obtain a quantitative measure of the level of the sensed constituent, such as ethanol, in the sampled stream.

A second preferred sensor embodiment is a semiconductor-type gas sensor, such as those utilizing a tin dioxide film as offered by Motorola, Figaro and other vendors. These sensors typically have a heated sensor element whose resistance varies in response to the level of organic vapors sensed.

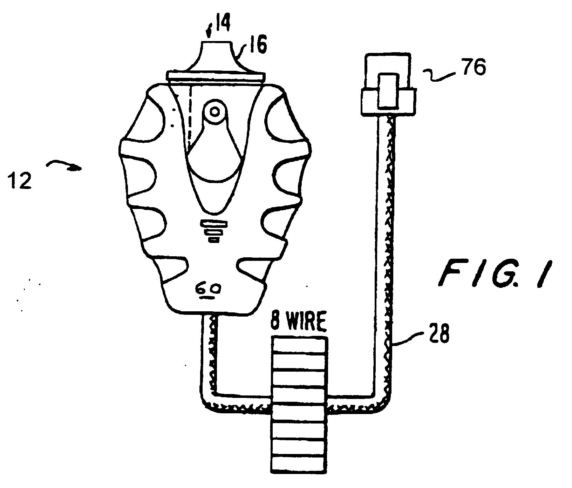

In accordance with the foregoing and the present invention, and as depicted in FIG. 1, a sensor head assembly 10 is mounted in a housing 12 which may be of compact design, capable of being held in the hand. Housing 12 has an input breath passageway 14 terminating at the exterior of the housing through nozzle 16. Preferably the nozzle 16 may be interchangeable to allow use of the device by a plurality of users. Sensing and associated circuitry is mounted within the housing, and are coupled to a remote processor through cable 28 and connector 76.

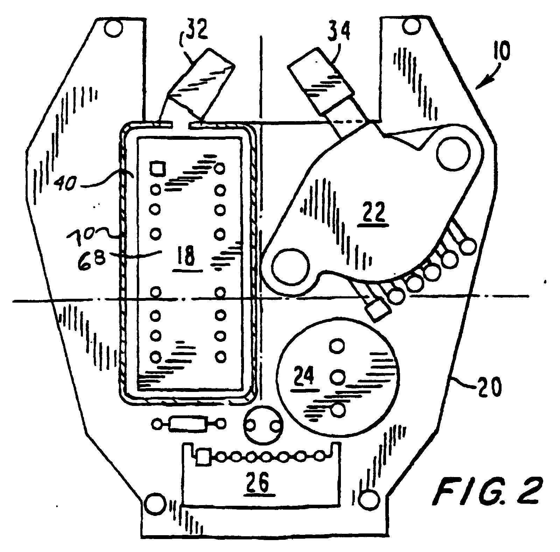

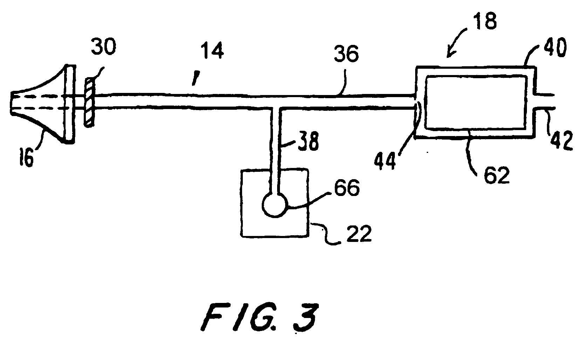

FIG. 3 presents the breath sample path for a first embodiment of the invention. As shown therein, the input breath passageway 14 is provided with a spit trap 30 proximate the nozzle 16 to trap excess breath moisture and to prevent such moisture from passing through the passageway to the sensing components. The spit trap may be in the form of a fine mesh across the passageway. The passageway then divides into first and second branch passageways 36, 38. The branch passageways may include discrete portions, such as coupling passageways 32, 34 depicted in FIG. 2, as well as portions formed with passageway walls integral with the housing 12, interconnecting the coupling passageways to input passageway 14. First branch passageway 36 leads to sensor module 18, which includes chamber 40 in which gas sensor 62 is located. The chamber is provided with an exhaust port or aperture 42 to permit a continuous flow of a breath sample to pass through the chamber and thus across the sensor within the chamber. In this embodiment the sensor is preferably a waveguide sensor system. The housing 12 is similarly vented to allow the breath sample to be exhausted to the ambient atmosphere.

Branch passageway 36 may be provided with a reduced diameter inlet aperture or port 44 located proximate the entrance to the chamber 40. The port creates a back pressure through the breath passageways leading to the chamber when a breath is applied, allowing such pressure to be sensed by a pressure transducer 22. The port may be, for example, 1.6 mm in diameter in a passageway system of 6.4 mm diameter. Alternatively, the geometry of the passageways themselves can provide sufficient backpressure effects to allow the pressure transducer to be operative. Chamber exhaust port 42 is of large diameter, which insures adequate evacuation of the chamber without development of significant additional backpressure therein and a continuing flow of breath therethrough. The volume of the chamber is such that it closely conforms to the geometry of the waveguide sensor system enclosed therein, providing a minimal volume allowing its contents to be replaced several times during the introduction of a breath sample.

The second branch passageway 38 leads to proportional pressure transducer 22. As known in the art, one side of the pressure-sensitive element of transducer 22 may define a portion of the interior surface of the transducer's internal sample reception chamber 66, while the opposite side of the pressure-sensitive element is exposed to ambient pressure within the housing. Pressure differences across the transducer generate an output electrical signal proportional to the pressure difference. For purposes of the present invention, the deviation in pressure of the breath sample over atmospheric may be in the range of .1 to .3 psi, and the pressure associated with a valid breath sample is chosen to be generally indicative of a sufficiently deep human breath which would include alveolar air.

As seen in FIG. 2, sensor head assembly 10 having the configuration of FIG. 3 may preferably include sensor module 18, which includes a sensor in the form of waveguide sensor 68 along with chamber 40 surrounding the sensing element into which the breath sample is directed for analysis. The waveguide sensor, which includes the associated drive and signal conversion circuits necessary to provide a variable frequency electrical signal output, is mounted to a printed circuit board 20. Also mounted to the board is the pressure transducer 22 and a sonic signalling device 24. A connector block 26 provides the necessary electrical connections between the elements and the cable 28, seen in FIG. 1, which couples the sensor head assembly to a remote processor. The sensor module 18 and the pressure transducer 22 are provided with associated coupling passageways 32, 34 forming respective portions of the branch passageways 36, 38 of FIG. 3 to provide the entry for a breath sample.

Chamber 40 may be formed as a cover-like assembly for sensor 68, affixed by mounting screws or the like to printed circuit board 20. Alternatively, it may be formed with its walls 70 being fabricated as by molding as internal walls in the interior of housing 12. The housing may be in a two-part construction, comprising a top 60, as seen in FIG. 1, and a mating bottom to facilitate construction of the internal walls and passageways, and to allow mounting of the printed circuit board therein. The chamber walls 70 project downwardly from the inner surface of top 60, and rest against the printed circuit board 20, surrounding waveguide sensor 68 to form the chamber 40. Coupling passageway 32 may be formed with coupling passageways 34 into an integral unit or alternatively can also be made integral with the housing 12.

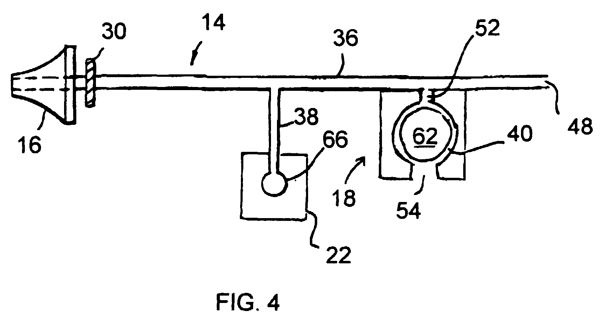

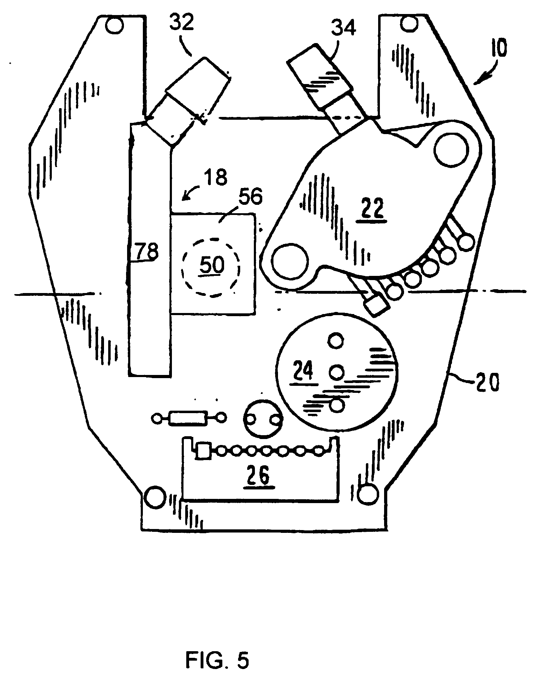

FIGs. 4-7 depict an alternative embodiment of the invention. With reference to FIGs. 4 and 5, the first branch passageway 36 is coupled to chamber 40 in a manner whereby only a portion of the breath sample in passageway 36 enters the chamber through aperture 52, the remaining majority of the sample exiting through exhaust aperture 48. The sample entering the chamber passes by sensor 62, which may be of the semiconductor type as designated by 50 in FIGs. 5-7, and exits through exhaust aperture 54. Thus two parallel paths for the breath are created in passageway 36, the first exiting directly through aperture 48 and the second through aperture 54 after passing past sensor 52/50.

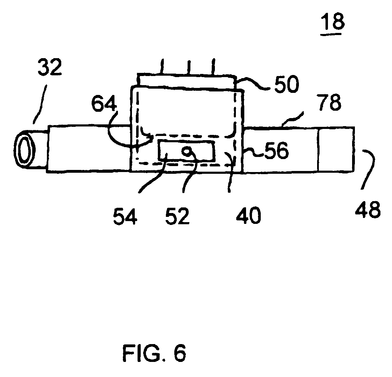

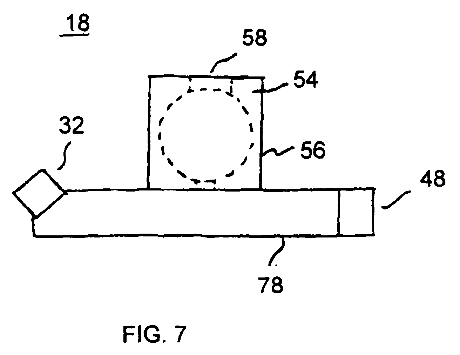

The sensor module 18 of the second embodiment, including chamber 40 and sensor 50, is detailed in FIGs. 6 and 7. As depicted therein, module 18 comprises chamber 40 as well as passageway portion 78 which corresponds to first branch passageway portion 32. Chamber 40 may be formed as a cavity within chamber housing 56 formed as a rectangular block. The cavity is cylindrical, and extends through face wall 58 of the block. The cavity diameter is chosen to accommodate the sensor 50, which is typically cylindrical, with the sample-receiving face 64 of the sensor being exposed within the chamber 40. The sidewall of sensor 50 forms an essentially air-tight friction fit with the accepting chamber cavity sidewall, thus negating the need for a separate chamber cover.

Inlet aperture 52 couples chamber 40 to the passageway portion 78, passing through the sidewall of the passageway portion and through the chamber housing, and is positioned such that the breath flow entering chamber 40 is directed across the exposed face 64 of the sensor 50. The passageway portion 78 may have an inner diameter on the order of .15 inch, inlet 52 being .02-.025 inch diameter. The exit aperture 54, formed through the distal face of the chamber housing block 56, is substantially larger, on the order of .075 inch by .150 inch, such that backpressure effects within the chamber are minimized, allowing the introduced gas sample to pass the sensor face in a continuing flow. The interior volume of the chamber is minimized by the presence of the sensor therein such that during an average expiratory breath the chamber's contents are replaced several times. This helps insure that the breath sample is not diluted by air originally in the chamber. Because the chamber is vented to the atmosphere through exit aperture 54, a portion of the increased pressure breath flow through the passageway system passes easily through inlet aperture 52, notwithstanding its small size. In the configuration of FIGs. 4-7, it has been found that no further restrictions in the flow path are required to generate sufficient back pressure within the system to allow pressure transducer 22 to operate.

In both embodiments power for the components within the housing 12 may be provided through the cable 28 affixed to connector 26. Connector 26 also leads the signals developed by the sensor module 18 and the pressure transducer 22 to an appropriate processor, which in the automotive environment may preferably be located behind or below the dashboard.

Sonic signalling element 24, also mounted on printed circuit board 20, is positioned to allow its signal to be perceived by the user. The sonic signalling element is intended to provide a feedback function to the user in connection with breath sampling, and to provide other aural indications associated with system operation. For example, it may provide a continuous tone during a sampling process to signify that the sample being received is proper. A particular tone pattern may be broadcast when breath sampling has been completed.

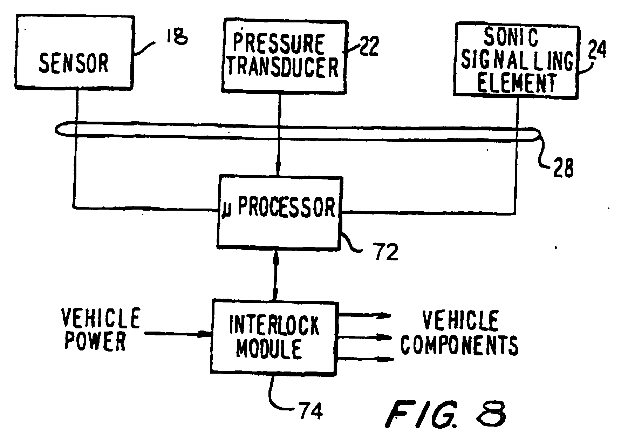

As seen in FIG. 8, sensor module 18, pressure transducer 22 and sonic signalling element 24 are coupled to remote processor 72 through the cable 28. The processor 72, which may be microprocessor based, is further coupled to interlock module 74 which serves as an interface to the vehicle, providing starter cut-off, power connections, and other necessary interconnections.

In operation, the user is prompted for a breath sample by the processor, either by the generation of a tone by the sonic signalling element or by another appropriate signal. The housing is brought to the mouth, the nozzle 16 being placed against the lips to allow the passageway 14 to be directly coupled to the mouth. The output of pressure transducer 22 reflects the internal pressure of the breath passageway 14, and is passed as an electrical signal to processor 72 which monitors the sensed pressure and activates sonic signalling element 24 for so long as the sensed pressure is above the baseline required for a valid breath sample, providing an indication to the user that the user is providing a proper breath. The sonic signalling element is activated for so long as a proper pressure is maintained, the processor timing the length of the breath to insure the generation of a valid sample necessary for sensor 18 to have an adequate breath volume for an accurate alcohol measurement. Such a time may be on the order of six seconds. It has been found that the combination of a pressure differential coupled with a several second flow through a passageway system as presented herein makes it extremely difficult for an artificial breath sample to be used in an effort to defeat the monitoring apparatus.

When the test period has successfully been completed, a double beep tone is generated by the sonic signalling element, advising the user that a valid breath sample has been received. If the continuous tone stops without a confirmation signal the user knows that the breath sample provided is not sufficient, and a new breath will be required. A new breath request signal can then be generated. The continuous tone thus provides oral feedback for the user, providing timing for the breath sample and confirming that he or she is providing a sufficiently deep breath for monitoring purposes. With receipt of a valid breath sample, processor 72 can accept for analysis the output of the sensor module 18, enabling the vehicle's starter circuit if the alcohol level is below a preset value, and/or generating other commands and signals as required or desired.

The breath sensor system of the present invention, utilizing an alcohol sensor having no moving parts, is of a highly rugged configuration yielding consistent results. Its high accuracy and ease of use, coupled with its small size, allow an efficient breath alcohol sensing system, as well as other sensing systems capable of detecting other breath constituents, to be employed.

## Claims

1. An apparatus for measuring the concentration of a human breath constituent comprising a hand-held housing; an internal breath passageway within said housing, said passageway having a first end extending through a nozzle mounted to the housing; a chamber within said housing coupled to said passageway; a sensor for detecting the level of the constituent mounted in said chamber and for providing an electrical signal output associated therewith; a pressure transducer coupled to said passageway for measuring the pressure therein and for providing an electrical signal output associated therewith; a sonic signalling element mounted in said housing for generation of indicator sounds perceptible outside the housing; and electrical coupling means in said housing for coupling said sensor, pressure transducer and sonic signalling element to a remote processor.

2. The apparatus of claim 1, wherein said chamber comprises a chamber wall formed integral with an interior portion of said housing.

3. The apparatus of claim 1 wherein said sensor is a sensor responsive to the presence of ethanol.

4. The apparatus of claim 3 wherein said sensor is a waveguide sensor.

5. The apparatus of claim 3 wherein said sensor is a semiconductor sensor.

6. The apparatus of claim 1 further including a backpressure-generating orifice in said passageway proximate said chamber.

7. The apparatus of claim 1 wherein said chamber comprises a chamber housing, an inlet aperture at a first end of the housing and an outlet aperture at a second end of the housing, said inlet aperture further comprising an aperture in a sidewall of the passageway to which the chamber is coupled.

8. The apparatus of claim 7 wherein the cross-sectional area of said inlet aperture is significantly smaller than the cross-sectional area of said outlet aperture.

9. The apparatus of claim 1 wherein said breath passageway includes first and second branch passageways, said pressure transducer coupled to said breath passageway by one of said branch passageways, said chamber coupled to said breath passageway by the other of said branch passageways.

10. The apparatus of clam 9 wherein the branch passageway coupling said chamber to said breath passageway has first and second parallel breath paths terminating in exit apertures, said second breath path comprising said chamber.

11. The apparatus of claim 11 wherein the chamber comprises a chamber housing, an inlet aperture at a first end of the housing and an said second breath path exit aperture at a second end of the housing.

12. The apparatus of claim 11 wherein said chamber comprises a cylindrical bore in the chamber housing extending through a face thereof, said sensor being frictionally mounted in said bore.
